(19) Europäisches Patentamt
European Patent Office
Office européen des brevets

(11) Veröffentlichungsnummer: **0 035 183**
**A2**

(12) # EUROPÄISCHE PATENTANMELDUNG

(21) Anmeldenummer: **81101199.8**

(22) Anmeldetag: **20.02.81**

(51) Int. Cl.³: **C 11 B 9/00**
// C07C69/00, A61K7/46

(30) Priorität: **27.02.80 DE 3007232**

(43) Veröffentlichungstag der Anmeldung: **09.09.81**
**Patentblatt 81/36**

(84) Benannte Vertragsstaaten: **AT BE CH DE FR GB IT LI LU NL SE**

(71) Anmelder: **Henkel Kommanditgesellschaft auf Aktien, -Patentabteilung- Postfach 1100 Henkelstrasse 67, D-4000 Düsseldorf 1 (DE)**

(72) Erfinder: **Upadek, Horst, Dr., Kempenweg 18 a, D-4006 Erkrath 2 (DE)**
Erfinder: **Conrad, Jens, Dr., Dürerweg 15, D-4010 Hilden (DE)**
Erfinder: **Bruns, Klaus, Dr., Notburgaweg 6, D-4150 Krefeld-Traar (DE)**

(54) **Verwendung von 2-Phenyl-1(2)-propen-1-yl-carbonsäureestern als Riechstoffe, deren Herstellung sowie diese enthaltende Riechstoffkompositionen.**

(57) Verwendung von 2-Phenyl-1(2)-propen-1-yl-carbonsäureestern der allgemeinen Formel

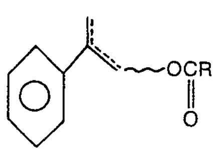

in der R eine Alkylkette mit 1–4 C-Atomen darstellt als Riechstoffe, diese enthaltende Riechstoffkompositionen und Herstellung der Verbindungen durch Umsetzung von 2-Phenyl-1,2-propandiol mit einem Überschuss eines entsprechenden Carbonsäureanhydrids in Gegenwart eines sauren Katalysators bei einer Temperatur von ca. 60 bis 125 °C.

EP 0 035 183 A2

HENKEL KGaA
ZR-FE/Patente
z-sü

P a t e n t a n m e l d u n g

D 6124 EP

"Verwendung von 2-Phenyl-1(2)-propen-1-yl-carbonsäureestern als Riechstoffe, deren Herstellung sowie diese enthaltende Riechstoffkompositionen"

Es wurde gefunden, daß 2-Phenyl-1(2)-propen-1-yl-carbonsäureester der allgemeinen Formel

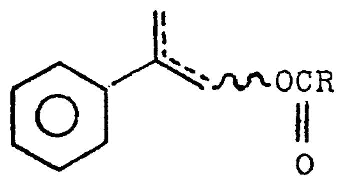

in der R eine Alkylkette mit 1 bis 4 Kohlenstoffatomen darstellt, in vorteilhafter Weise als Riechstoffe mit kräftiger, blumiger Geruchsnote verwendet werden können.

Die Herstellung der erfindungsgemäß als Riechstoffe zu verwendenden 2-Phenyl-1(2)-propen-1-yl-carbonsäureester erfolgt durch säurekatalysierte Umsetzung von 2-Phenyl-1,2-propandiol mit überschüssigem Carbonsäureanhydrid unter Dehydratisierung der tertiären Hydroxylgruppe und gleichzeitiger Veresterung der primären Hydroxylgruppe gemäß nachfolgendem Reaktionsschema. Das als Ausgangsmaterial zur Herstellung der Ester dienende 2-Phenyl-1,2-propandiol ist technisch leicht und preiswert aus α-Methylstyrol zugänglich.

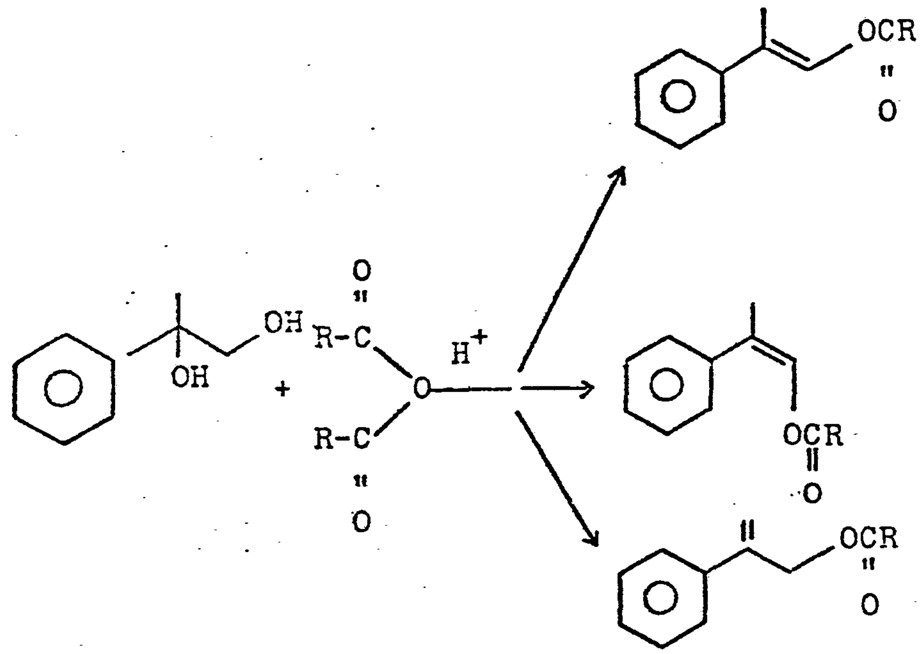


Das auf diese Weise erhaltene Gemisch der drei isomeren Ester entspricht dem erfindungsgemäß zu verwendenden 2-Phenyl-1(2)-propen-1-yl-carbonsäureester vorgenannter allgemeiner Formel und kann ohne weitere Auftrennung in die einzelnen Komponenten als Riechstoff verwendet werden.

Zur vollständigen Umsetzung des 2-Phenyl-1,2-propandiols zu den gewünschten Estern sind der Einsatz von etwa 3 Mol Carbonsäureanhydrid pro Mol Diol sowie erhöhte Reaktionstemperaturen von ca. 60 bis 125° C empfehlenswert. Als Katalysatoren sind z. B. Schwefelsäure, p-Toluolsulfonsäure oder saure Festbettkatalysatoren geeignet. Das Isomerenverhältnis der gebildeten 2-Phenyl-1(2)-propen-1-yl-carbonsäureester schwankt mit Variation der Reaktionsbedingungen, wie z. B. Variation der Anhydridmenge, der Reaktionszeit und der Reaktionstemperatur. Ein wesentlicher Einfluß des Isomerenverhältnisses des 2-Phenyl-1(2)-propen-1-yl-carbonsäureesters auf dessen Geruchseigenschaften ist nicht erkennbar.

Besondere Bedeutung kommt dem aus 2-Phenyl-1,2-propandiol und Acetanhydrid erhältlichen 2-Phenyl-1(2)propen-1-yl-acetat mit seiner süß-blumigen, an Flieder, Hyazinthen und reifes Getreide erinnernden Geruchsnote zu. Dieser Ester eignet sich z. B. hervorragend zur Formulierung interessanter Neroli- und Fliederkompositionen.

Das 2-Phenyl-1(2)-propen-1-yl-acetat ist als Produkt literaturbekannt und auf den verschiedensten Wegen hergestellt worden, ohne daß seine Verwendungsmöglichkeit und Bedeutung als Riechstoff, beziehungsweise Komponente von Riechstoffkompositionen erkannt worden wäre. So beschreiben A. Wohl und E. Berthold in Ber. 43, 2184 (1910) die Herstellung aus Hydratropaldehyd und Essigsäureanhydrid in Gegenwart von Salzsäure K. v. Auwers in Ann. 413, 280 (1917) die Herstellung aus Hydratropaldehyd und Essigsäureanhydrid in Gegenwart von Natriumacetat. Eine Reihe weiterer Herstellungsverfahren ausgehend von α-Methylstyrol wird in neuerer Zeit beschrieben, z. B. von A. Kasahara, R. Saito und T. Izmi, Bull. Chem. Soc. Jap. 46 (1973), 2610 und in der belgischen Patentschrift 630 862 der Firma General Electric Co.

Die Herstellung der erfindungsgemäß zu verwendenden Riechstoffe erfolgt durch Umsetzung von 2-Phenyl-1,2-propandiol mit Essigsäureanhydrid, Propionsäureanhydrid, Buttersäureanhydrid und Isovaleriansäureanhydrid in Gegenwart saurer Katalysatoren.

Als erfindungsgemäß zu verwendende 2-Phenyl-1(2)-propen-1-yl-carbonsäureester sind z. B. 2-Phenyl-1(2)-propen-1-yl-acetat, -propionat, -butyrat und -3-methylbutyrat zu nennen.

Die erfindungsgemäß zu verwendenden 2-Phenyl-1(2)-propen-1-yl-carbonsäureester stellen wertvolle Riechstoffe mit kräftigen blumigen Geruchsnoten dar. Ein besonderer Vorteil ist ihre sehr gute Kombinationsfähigkeit zu interessanten Geruchsnuancen.

Die erfindungsgemäß zu verwendenden 2-Phenyl-1(2)-propen-1-yl-carbonsäureester können mit anderen Riechstoffen in den verschiedensten Mengenverhältnissen zu neuen Riechstoffkompositionen gemischt werden. Im allgemeinen wird sich jedoch der Anteil der erfindungsgemäß zu verwendenden 2-Phenyl-1(2)-propen-1-yl-carbonsäureester in den Riechstoffkompositionen in den Mengen von 1 bis 50 Gewichtsprozent, bezogen auf die gesamte Komposition, bewegen. Derartige Kompositionen können direkt als Parfüm oder zweckmäßigerweise auch zur Parfümierung von Kosmetika wie Cremes, Lotionen, Duftwässern, Aerosolen, Toiletteseifen usw. dienen. Sie können aber auch zur Geruchsverbesserung technischer Produkte wie Wasch- und Reinigungsmittel, Desinfektionsmittel, Textilbehandlungsmittel usw. eingesetzt werden.

Die nachfolgenden Beispiele sollen den Gegenstand der Erfindung näher erläutern, ohne ihn jedoch hierauf zu beschränken.

## Beispiele

Zunächst wird die Herstellung der erfindungsgemäß zu verwendenden 2-Phenyl-1(2)-propen-1-yl-carbonsäureester beschrieben.

### 2-Phenyl-1(2)-propen-1-yl-acetat

Zu 15,2 g (0,1 Mol) 2-Phenyl-1,2-propandiol und 30,6 g (0,3 Mol) Acetanhydrid wurden unter Rühren und Kühlung ein bis zwei Tropfen konzentrierter Schwefelsäure zugegeben. Die Temperatur der Reaktionslösung stieg durch das Einsetzen der stark exothermen Reaktion auf 85° C. Es wurde anschließend 2 Stunden bei 125° C nachgerührt. Überschüssiges Acetanhydrid und gebildete Essigsäure wurden abdestilliert, die Reaktionsmischung mit 20 %iger Natriumcarbonatlösung neutralisiert und mit Ether extrahiert. Nach Trocknen über Magnesiumsulfat und Entfernen des Lösungsmittels wurde im Vakuum destilliert. Es wurden 12,7 g (86 % der Theorie) an 2-Phenyl-1(2)-propen-1-yl-acetat vom Kp. 94-96° C/2,7 mbar und Brechungsindex $n_D^{20}$ = 1,5324 erhalten. Das Produkt besitzt einen süßen, blumigen Duft nach Flieder, Hyazinthe und reifem Getreide.

### 2-Phenyl-1(2)-propen-1-yl-propionat

Die Herstellung erfolgte völlig analog dem Acetat und ergab ein Produkt vom Kp. 129 - 131° C/4 mbar und einem Brechungsindex $n_D^{20}$ = 1,5211. Der Geruch ist dem des 2-Phenyl-1(2)-propen-1-yl-acetats sehr ähnlich.

/6

2-Phenyl-1(2)-propen-1-yl-butyrat

Auch diese Herstellung erfolgte völlig analog aus 2-Phenyl-1,2-propandiol und Buttersäureanhydrid und lieferte ein Produkt vom Kp 78-80°C/0,01 mbar, und einem Brechungsindex $n_D^{20}$= 1,5193. Der Geruch ist dem des 2-Phenyl-1(2)-propen-1-yl-acetats ähnlich, jedoch herber.

Nachfolgend wird noch ein Kompositionsbeispiel angeführt:

Hyazinthe-Komplex

| | | |
|---|---|---|
| 2-Phenyl-1(2)-propen-1-yl-acetat | 150 | Gewichtsteile |
| Phenylethylalkohol | 200 | " |
| Benzylsalicylat | 200 | " |
| Hydroxycitronellal | 120 | " |
| Linalool | 100 | " |
| α-Hexylzimtaldehyd | 60 | " |
| Phenylacetaldehyd-dimethylacetal | 50 | " |
| Geraniol natürlich | 30 | " |
| Ylang-Ylang-Öl | 30 | " |
| Phenylethylacetat | 20 | " |
| Galbanum Res. synth. | 20 | " |
| Cyclamenaldehyd | 10 | " |
| Styrallylacetat | 10 | " |
| | 1 000 | Gewichtsteile |

"Verwendung von 2-Phenyl-1(2)-propen-1-yl-carbonsäureestern als Riechstoffe, deren Herstellung sowie diese enthaltende Riechstoffkompositionen"

Patentansprüche:

1. Verwendung von 2-Phenyl-1(2)-propen-1-yl-carbonsäureestern der allgemeinen Formel

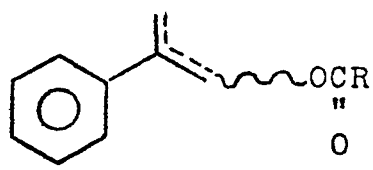

in der R eine Alkylkette mit 1 bis 4 Kohlenstoffatomen darstellt, als Riechstoffe.

2. Verwendung von 2-Phenyl-1(2)-propen-1-yl-carbonsäureestern nach Anspruch 1, dadurch gekennzeichnet, daß in der genannten allgemeinen Formel R einen Methylrest darstellt.

3. Riechstoffkompositionen, gekennzeichnet durch einen Gehalt an 2-Phenyl-1(2)-propen-1-yl-carbonsäureestern nach Anspruch 1 und 2.

4. Riechstoffkompositionen nach Anspruch 3, dadurch gekennzeichnet, daß sie die 2-Phenyl-1(2)-propen-1-yl-carbonsäureester in einer Menge von 1 bis 50 Gewichtsprozent, bezogen auf die gesamte Komposition, enthalten.

5. Verfahren zur Herstellung der gemäß Ansprüche 1 - 4 einzusetzenden 2-Phenyl-1(2)-propen-1-yl-carbonsäureester, dadurch gekennzeichnet, daß man 2-Phenyl-1,2-propandiol mit einem Überschuß eines Carbonsäureanhydrids der Formel $(RCO)_2O$, in der R eine Alkylkette mit 1 bis 4 Kohlenstoffatomen darstellt, in Gegenwart eines sauren Katalysators bei einer Temperatur von ca. 60 - 125° C umsetzt und das Reaktionsgemisch in üblicher Weise aufarbeitet.